# EUROPEAN PATENT SPECIFICATION

(11) **EP 1 889 628 B1**
(45) Date of publication and mention of the grant of the patent: **11.12.2013**
(21) Application number: 06746576.5
(22) Date of filing: 11.05.2006
(51) Int. Cl.: A61K 38/47, A61K 9/133, A61K 47/18, A61P 3/06, A61K 9/127, A61K 9/00

(54) **LIPID LIPOSOME COMPOSITION**
LIPID-LIPOSOM-ZUSAMMENSETZUNG
COMPOSITION DE LIPOSOMES LIPIDIQUES

(30) Priority: 11.05.2005 JP 2005139174
(43) Date of publication of application: 20.02.2008
(73) Proprietor: JCR PHARMACEUTICALS Co., LTD., Ashiya, Hyogo 659-0021 (JP); Tokyo Metropolitan Institute of Medical Science, Tokyo (JP)
(72) Inventor: SAKURABA, Hitoshi, 2701164 (JP); TAJIMA, Youichi, 1210815 (JP); KAWASHIMA, Ikuo, 3650064 (JP); ITO, Mai, 1350047 (JP); TAKEOKA, Shinji, 1580094 (JP); OHTA, Katsuji, 3330862 (JP); ITO, Manabu, 1350047 (JP); MIZUNO, Atsushi, Meguro-ku, Tokyo 1520035 (JP)
(74) Representative: Denison, Christopher Marcus
(86) International application number: PCT/JP2006/309878
(87) International publication number: WO 2006/121199

(56) References cited:
- JP-A- 2003 513 901
- JP-A- 2004 269 442
- DAS P K ET AL: "Lectin-specific targeting of beta-glucocerebrosidase to different liver cells via glycosylated liposomes", BIOCHEMICAL MEDICINE, ACADEMIC PRESS, SAN DIEGO, CA, US, vol. 33, no. 1, 1 February 1985 (1985-02-01), pages 124-131, XP026166134, ISSN: 0006-2944, DOI: 10.1016/0006-2944(85)90135-8 [retrieved on 1985-02-01]
- K. SOU ET AL: "Effective Encapsulation of Proteins into Size-Controlled Phospholipid Vesicles Using Freeze-Thawing and Extrusion", BIOTECHNOLOGY PROGRESS, vol. 19, no. 5, 3 October 2003 (2003-10-03), pages 1547-1552, XP55043627, ISSN: 8756-7938, DOI: 10.1021/bp0201004
- HIROMI SAKAI ET AL: "O2 release from Hb vesicles evaluated using an artificial, narrow O2-permeable tube: comparison with RBCs and acellular Hbs", AM J PHYSIOL HEART CIRC PHYSIOL, 24 July 2003 (2003-07-24), pages 2543-2551, XP55043624, DOI: 10.1152/ajpheart.00537.2003
- PIJUSH K. ET AL.: 'Lectin-Specific Targeting of beta-Glucocerebrosidase to Different Liver Cells via Glycosylated Liposomes' BIOCHEMICAL MEDICINE vol. 33, no. 1, 1985, pages 124 - 131, XP008072494

## Description

### TECHNICAL FIELD

The present invention relates to a lipid vesicle composition in which an enzyme is encapsulated in the internal aqueous phase of the lipid vesicle.

### BACKGROUND ART

Lysosomal disease is a hereditary disease caused by activity decrease or defects in lysosomal enzymes and their related factors and by the resultant storage of the substrates of such enzymes in the living body. For example, in Gaucher disease that is one type of lysosomal disease, activity decrease in glucocerebrosidase (β-glucosidase) causes storage of glucocerebroside in cells such as macrophages in reticuloendothelial tissues. As a result, symptoms and observations such as splenohepatomegaly; anemia and decreased platelet count associated with enhancement of splenic function; bone lesions; increase in the levels of blood acidic phosphatase and angiotensin converting enzyme; and the like are observed.

As a method for treating lysosomal disease, enzyme replacement therapy has been frequently adopted. In Gaucher disease, for example, a recombinant enzyme expressed by using a cDNA encoding human glucocerebrosidase in a Chinese hamster ovary (CHO) cell strain is used with a saccharide-chain modified form so that to be uptaken into target cell macrophages easily (e.g., mannose residues are added at the non-reducing terminal of the enzyme in order to facilitate recognition by the mannose receptor present on the surface of target cell macrophages).

However, in enzyme replacement therapy, the stability in blood (blood residence) and the transfer to target organs (targeting property (transfer-to-target property; potential target transfer)) of enzyme preparations have not been sufficient. Even those enzyme preparations which are saccharide-chain modified as described above to give or improve targeting property are still insufficient.

Therefore, enzyme replacement therapy becomes a highly restricting therapy; e.g., for the treatment of Gaucher disease (especially, type I), glucocerebrosidase is administered to patients by intravenous infusion "for 1-2 hours/administration, 60 units/kg body weight and at intervals of two weeks". In any of the administration time, dose and intervals, this therapy is highly restricting. Furthermore, the total dose becomes inevitably -high. Therefore, physical and economical burden to patients has become a serious problem.

On the other hand, liposome compositions encapsulating glucocerebrosidase that is closely related to Gaucher disease have already been known (ISOBEL P. BRAIDMAN et al., Rapid Partial Purification of Placental Glucocerebroside β-Glucosidase and its Entrapment in Liposomes, Biochem. J., vol. 164, 1977, pp. 439-445; Gregory Gregoriadis et al., Experiences After Long-Term Treatment of a Type I Gaucher Disease Patient With Liposome-Entrapped Glucocerebroside: β-Glucosidase, "ENZYME THERAPY IN GENETIC DISEASES: 2", ALAN R. LISS, INC., NEW YORK, 1980, pp. 383-392; Gregory Gregoriadis et al., LIPOSOMES IN GAUCHER TYPE I DISEASE: USE IN ENZYME THERAPY AND THE CREATION OF AN ANIMAL MODEL, "GAUCHER DISEASE: A CENTURY OF DELINEATION AND RESEARCH", ALAN R. LISS, INC., NEW YORK, 1982, pp. 681-701). However, any of these liposome compositions have not been contemplated so that the enzyme activity is sufficiently stably retained in plasma (*in vitro*) or in blood flow (*in vivo*). Therefore, it can be easily presumed that they would be as poor as conventional enzymes in both blood residence and targeting property when they are used as enzyme preparations in enzyme replacement therapy.

DAS P K ET AL: "Lectin-specific targeting of beta-glucocerebrosidase to different liver cells via glycosylated liposomes", BIOCHEMICAL MEDICINE, ACADEMIC PRESS, SAN DIEGO, CA, US, vol. 33, no. 1, 1 February 1985 (1985-02-01), pages 124-131, discloses bilayer lipid vesicles comprising specific sugars exposed on the bilayer to deliver of glucocerebrosidase (a lysosomal enzyme) to non-parenchymal cells of liver. The liposomes are obtained in the presence of PBS buffer.

### DISCLOSURE OF THE INVENTION

It is an object of the present invention to provide an enzyme preparation which is excellent in stability in blood (blood residence) and in transfer to target organs (targeting property) and can be used effectively in enzyme replacement therapy and the like.

In the process of extensive and intensive researches to solve the above problems, the present inventors have encapsulated an enzyme of interest in vesicles composed of a lipid bilayer membrane and focused on handling the vesicles under such a state that the original activity of the encapsulated enzyme is sufficiently retained even when the vesicles are placed outside the pH-range of the enzyme kept stable. As a result, the present inventors have succeeded in greatly enhancing the stability in blood and the targeting property of an enzyme compared to the enzyme itself and conventional encapsulated enzymes in vesicles. When this lipid vesicle composition is used in enzyme replacement therapy, it is believed that decrease of administration time (dose/administration) and prolongation of administration intervals can be effectively achieved. As a result, burden to patients will be greatly reduced or improved, and that will be able to contribute to improve the patients' quality of life greatly. The present invention has been achieved as described above.

The present invention relates to the following.
(1) A lipid vesicle composition wherein vesicles composed of a lipid bilayer membrane encapsulate therein a lysosomal enzyme relating to lysosomal disease,
   wherein the lipid bilayer membrane contains diacylphosphatidylcholine, cholesterol, 1,5-O-dihexadecyl- *N*-succinyl-L-glutamateand a polyethylene glycol (PEG) chain-linked lipid, wherein the molecular weight of the PEG chain is 2000-5000, and
   wherein the lysosomal enzyme is in aqueous solution including a pH buffer, wherein the pH of the aqueous solution is within the stable pH range of the enzyme.
   As a specific example of the above enzyme, glucocerebrosidase may be given.
(2) The lipid vesicle composition of (1) above in which glucocerebrosidase is encapsulated in the vesicles may be used as a therapeutic agent for Gaucher disease.
(3) A method for stabilizing a lysosomal enzyme, comprising encapsulating the enzyme in vesicles composed of the lipid bilayer membrane of (1), in the presence of a pH buffer and optionally in the presence of a surfactant.
   As a specific example of the enzyme, glucocerebrosidase may be given.
(4) A method for preparing the lipid vesicle composition of (1), comprising encapsulation of a lysosomal enzyme in vesicles composed of a lipid bilayer membrane in the presence of a pH buffer and optionally in the presence of a surfactant.
   In the above method, it is possible to allow the pH buffer and/or the surfactant to be present in both the internal aqueous phase and the external aqueous phase of the vesicles formed.
   As a specific example of the above enzyme, glucocerebrosidase may be given.
(5) A method for treating Gaucher disease, comprising administration of a Gaucher disease patient the composition of (1) above containing glucocerebrosidase.

### BRIEF DESCRIPTION OF THE DRAWINGS

Fig. 1 shows the change of the enzyme activity in plasma after administration of glucocerebrosidase in a free form and in a liposome preparation.
Fig. 2A to Fig. 2C shows the enzyme activity in each tissue after administration of glucocerebrosidase in a free form and in a liposome preparation. Fig. 2A shows the enzyme activity in the spleen; Fig. 2B shows the enzyme activity in the liver; and Fig. 2C shows the enzyme activity in the kidney.
Fig. 3 shows the results of Western blotting of splenic samples after administration of glucocerebrosidase in a free form or in a liposome preparation.
Fig. 4 shows the stability of glucocerebrosidase in a free form or in a liposome preparation in human plasma (*in vitro*).
Fig. 5 is photographs of immunostained glucocerebrosidase (green) and Kupffer cells (red) in the liver after administration of glucocerebrosidase in a free form or in a liposome preparation.

### BEST MODES FOR CARRYING OUT THE INVENTION

Hereinbelow, the present invention will be described more specifically. However, the scope of the present invention is not limited to the illustrations to be given below, and the present invention may be practiced by variously modifying the illustrations below without departure from the spirit of the present invention.

### 1. Lipid Vesicle Composition

### (1) Lipid Vesicle

As the lipid which constitutes the membrane of a vesicle, one or more lipids selected from diacylphosphatidylcholines are used such as dimyristoylphosphatidylcholine, dipalmitoylphosphatidylcholine, distearoylphosphatidylcholine, and dilinoleoylphosphatidylcholine; diacylphosphatidic acid; diacylphosphatidylethanolamine; diacylphosphatidylglycerol; diacylphosphatidylinosytol; and sphingophospholipids. Among them, dimyristoylphosphatidylcholine, dimyristoylphosphatidylcholine and distearoylphosphatidylcholine are more preferable.

When a combination of a saturated phospholipid and an unsaturated phospholipid are used in combination, examples of preferable combinations include, but are not limited to, combinations of a synthetic saturated phospholipid (such as dipalmitoylphosphatidylcholine or distearoylphosphatidylcholine) and a synthetic unsaturated phospholipid (such as 1-palmitoyl-2-oleoylphosphatidylcholine or dioleoylphosphatidylcholine). Among them, a combination of dipalmitoylphosphatidylcholine and 1-palmitoyl-2-oleoylphosphatidylcholine is preferable.

The lipid constituting the membrane of a vesicle includes a polyethylene glycol (PEG)-type lipid (i.e., PEG chain-linked lipid). By containing a PEG-type lipid, the lipid vesicle formed has PEG chains on its surface. As a result, changes in particle size caused by vesicle aggregation or fusion when dried vesicle particles are resuspended in an aqueous solution or during a period of storage can be suppressed effectively. Decrease in filter permeability and clogging of filter can be prevented when extrusion method is performed for particle size control. Furthermore, hemodynamics in blood can be effectively improved.

When the vesicle contains a PEG-type lipid, the ratio of the PEG-type lipid is not particularly limited. The ratio is preferably 0.01-10 mol%, more preferably 0.1-1 mol% and still more preferably 0.1-0.3 mol%, relative to the total lipid constituting the membrane. When the ratio of the PEG-type lipid is below the above-described range, the effects that inhibit aggregation and improve hemodynamics provided by PEG chains are likely to become weak. When the ratio of the PEG-type lipid is beyond the above-described range, encapsulation efficiency of the substance contained in the internal aqueous phase of the vesicle is likely to decrease because of the exclusion volume effect of PEG chains extended into the internal aqueous phase, and unnecessary PEG chains are likely to lead cost increase.

The molecular weight of the PEG chain in the PEG-type lipid is 2,000-5,000. When the molecular weight of the PEG chain is in the above range, the above-described suppression effects on particle size change and aggregation can be obtained more effectively.

The lipid constituting the membrane of a vesicle may include a saccharide-chain (such as polysaccharide)-linked lipid. By containing such a lipid, the lipid vesicle formed has saccharide-chains on its surface. As a result, transfer of the vesicle to a target cell, tissue or organ can be improved further. Saccharide-chains to be used for this purpose are not particularly limited. For example, polysaccharides with mannose or mannose 6-phosphate at their terminals may be used.

When such a saccaride-chain linked lipid is contained, the ratio of this lipid is not particularly limited. The ratio is preferably 0.01-10 mol%, more preferably 0.1-1 mol%, relative to the total lipid constituting the membrane from the viewpoint of achieving the above-described improvement of targeting property more effectively.

The lipid constituting the membrane of a vesicle may include a negatively charged lipid (anionic lipid). Examples of negatively charged lipids include, but are not limited to, diacylphosphatidylglycerol, diacylphosphatidic acid, diacylphosphatidylinositol, diacylphosphatidylserine and fatty acids. Examples of fatty acids include, but are not limited to, C₁₂-₂₀ saturated or unsaturated fatty acids; more specifically, myristic acid, palmitic acid, stearic acid, oleic acid, linoleic acid, linolenic acid, octadeca-2,4-dienoic acid or the like may be enumerated.

When a negatively charged lipid is contained, the ratio of this lipid is not particularly limited. The ratio is preferably 1-50 mol%, more preferably 5-20 mol%, relative to the total lipids constituting the membrane. When the ratio is below the above-mentioned range, the entrapment efficiency of encapsulated substance is likely to decrease. When the ratio is above the above-mentioned range, the membrane of a vesicle is likely to become unstable.

Furthermore, the lipid constituting the membrane of a vesicle contains cholesterol as a stabilized.

The ratio of cholesterol should be 5-50 mol%, preferably 15-40 mol%, relative to the total lipid constituting the membrane in order to effectively stabilize the vesicle membrane.

With respect to the lipid used as a stabilizer, an anionic lipid is also used. Homocysteine or glutathione may also be used.

The anionic lipid is the carboxylic acid-type lipid DHSG (1,5-*O*-dihexadecyl-*N*-succinyl-L-glutamate) represented by the formula below.

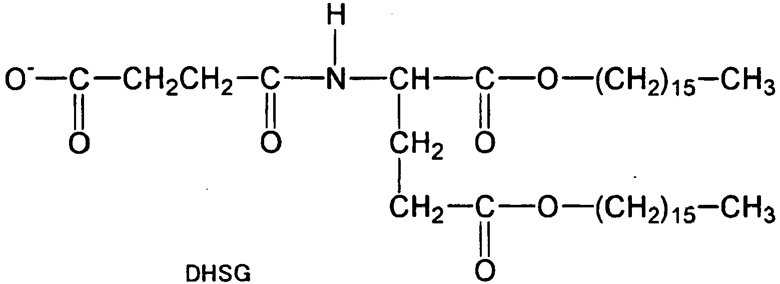

### (2) Enzyme as a Substance to be Encapsulated

The enzyme to be encapsulated in the lipid vesicle is a lysosomal enzyme relating to lysosomal disease.

Examples of such lysosomal enzymes include those shown in the rightmost column in Table 1 below. Among all, glucocerebrosidase (β-glucosidase) associated with Gaucher disease is preferable.

**Table 1**

| Major Lysosomal Diseases | | | | |
|---|---|---|---|---|
| Disease | | | Deficient Enzyme | Major Accumulated Substance |
| 1) Sphingolipidosis | | | | |
| | G_{M1} gangliosidosis | | β-Galactosidase | G_{M1} ganglioside; Oligosaccharides and glycoproteins with β-galactose residue |
| | G_{M2} ganglioshidosis | | | |
| | B variant (Tay-Sachs disease) | | β-Hexosaminidase A | G_{M2} ganglioside |
| | Q variant (Sandhoff disease) | | β-Hexosaminidase A and B | G_{M2} ganglioside; G_{A2} ganglioside; Oligosaccharides and glycoproteins with β-N-acetylglucosamine residue |
| | Fabry disease | | α-Galactosidase | Globotriaosylceramide |
| | Metachromatic leukodystrophy | | Arylsulfatase A | Sulfatide |
| | Krabbe disease (Globoid cell leukodystrophy) | | Galactocerebrosidase | Galactocerebroside, Psychosine |
| | Gaucher disease | | Glucocerebrosidase | Glucocerebroside |
| | Niemann-Pick disease (type A and type B) | | Acid sphingoemyelinase | Sphingomyelin |
| | Farber disease | | Acid ceramidase | Ceramide |
| | | | | |
| 2) Cholesterol ester and triglyceride storage disease | | | | |
| | Wolman and cholesterol ester storage disease | | Acid lipase | Cholesterol ester and triglyceride |
| | | | | |
| 3) Glycogenesis | | | | |
| | Pompe disease | | Acid α-glucosidase (acid maltase) | Glycogen |
| 4) Glycoprotein storage disease | | | | |
| | Fucosidosis | | α-Fucosidase | Oligosaccharides and glycoproteins with α-fucose residue |
| | α-Mannosidosis | | α-Mannosidase | Oligosaccharides and glycoproteins with α-mannose residue |
| | β-Mannosidosis | | β-Mannosidase | Oligosaccharides and glycoproteins with β-mannose residue |
| | Sialidosis | | Lysosomal neuraminidase | Oligosaccharides and glycoproteins with sialic acid residue |
| | Aspartylglucosaminuria | | Aspartylglucosaminidase | Aspartylglucosamine |
| | Schindler disease and Kanzaki disease | | α-N-acetylgalactosaminidase | O-linked oligosaccharides and glycoproteins with α-N-acetylgalactosamine residue |
| 5) Mucopolysaccharidoses | | | | |
| | Type I (Hurler, Scheie) | | α-Iduronidase | Dermatan sulfate; heparan sulfate |
| | Type II (Hunter) | | Iduronate sulfatase | Dermatan sulfate; heparan sulfate |
| | Type III (Sanfilippo) | A | Heparan N-sulfatase | Heparan sulfate |
| | | B | α-N-acetylglucosaminidase | Heparan sulfate |
| | | C | Acetyl CoA: α-glucosaminido acetyl transferase | Heparan sulfate |
| | | D | N-acetylglucosamine 6-sulfatase | Heparan sulfate |
| | Type IV (Morquio) | A | Galactose 6-sulfatase | Ketaran sulfate; chondroitin 6-sulfate |
| | | B | β-Galactosidase | Ketaran sulfate |
| | Type VI (Maroteaux-Lamy) | | Arylsulfatase B | Dermatan sulfate |
| | Type VII (Sly) | | β-Glucuronidase | Dermatan sulfate; hepatan sulfate; chondroitin 4-, 6-sulfate |

The enzyme *per se* to be encapsulated in the lipid vesicle may be saccharide-chain-modified. However, in the present invention, enzymes without saccharide chain modification are also enumerated as preferable embodiments.

Generally, in conventional enzyme replacement therapy, presence of a saccharide (a ligand for the receptor on a target cell) at the non-reducing terminal of the enzyme saccharide-chain is known as a preferable embodiment to enhance the enzyme's targeting property. Preferable examples of saccharide-chain modified enzymes include mannose-linked enzymes such as glucocerebrosidase and enzymes in which mannose 6-phosphate is linked to the non-reducing terminal of β-galactosidase saccharide-chains.

However, in the present invention, since the enzyme to be supplemented goes through an uptake process by a target cell in the state of encapsulation in the lipid vesicle, the enzyme can be sufficiently supplemented to the target cell even if the enzyme *per se* has not be given any improvement to enhance its targeting property Therefore, in the present invention, even when the enzyme is not saccharide-chain modified, various effects can be expected, e.g., enzyme productivity (enzyme efficiency) will be improved and the original activity of the enzyme can be expressed more easily. Thus, still higher treatment effect can be expected when such an enzyme is used in enzyme replacement therapy or the like.

Usually, an enzyme is encapsulated in a lipid vesicle in the state of an aqueous enzyme solution. The aqueous medium contained in the aqueous solution is capable of retaining the activity of the encapsulated enzyme in a stable state (i.e., state in which the original activity of the enzyme or an activity equivalent thereto can be expressed), a pH buffer. As the pH buffer, glycine buffer, monopotassium phthalate buffer, succinate buffer, monopotassium citrate buffer, Tris(hydroxymethyl)aminomethane buffer, borate buffer, acetate buffer or the like may be enumerated. When the enzyme is glucocerebrosidase, monopotassium citrate buffer is preferable.

The pH of the above-mentioned aqueous solution is appropriately adjusted or set within the stable pH range of the enzyme to be encapsulated. For example, when the enzyme is glucocerebrosidase, the pH range of the aqueous solution is preferably 2 to 7, more preferably 4 to 6.5. Some of the enzyme preparations (enzyme *per se*, etc.) used in enzyme replacement therapy to treat lysosomal diseases have shown remarkable activity decrease before they are transferred to their target cells. It is believed that this activity decrease during transfer occurred mainly because the pH (neutral range) in blood flow (plasma) is outside the stable pH range for the activity of the enzyme. Therefore, when an enzyme is encapsulated in a lipid vesicle while maintaining the condition of stable pH range as described above, it is possible to stabilize the enzyme activity during transfer to thereby improve the targeting property of the enzyme eventually.

The enzyme concentration in the above-described aqueous enzyme solution (i.e., enzyme concentration in the internal aqueous phase of the composition of the present invention) is not particularly limited. The concentration may be appropriately determined with consideration of the type of the relevant enzyme, the use of the composition, the progress of the target disease, etc.

### (3) Other Components

The lipid vesicle composition of the present invention may comprise other components appropriately in addition to the above-described lipid vesicle and the enzyme (aqueous enzyme solution) encapsulated therein. The other components are not particularly limited.

Examples of other components include, but are not limited to, amino acids, saccharides, reducing agents, vitamins, bases and the like to be contained in the internal aqueous phase of the lipid vesicle.

### (4) Method for Preparing Lipid Vesicle Composition

The method for preparing the lipid vesicle composition of the present invention is characterized by encapsulating an enzyme in vesicles composed of a lipid bilayer membrane in the presence of a pH buffer and/or a surfactant.

In the preparation method of the present invention, the encapsulation of enzymes in a vesicle composed of a lipid bilayer membrane may be performed by known, common method which is used in preparing lipid vesicles. For example, the vortex method, ultrasonic irradiation, high pressure output, high pressure extrusion, forced agitation (the homogenizer method), freeze-thawing, organic solvent injection, surfactant removal, reversed phase evaporation or the microfluidizer method may be used alone or in a combination. Means and conditions for practicing these methods may be appropriately selected and set based on the technical commonsense of those skilled in the art.

Specifically, a desired lipid to form a membrane (generally, in a powder form) is added to an aqueous solution of enzymes to be encapsulated, to thereby make the lipid hydrated or swollen. The resultant mixture is left to stand and then treated with a vortex mixer, forced agitator, ultrasonic irradiator (such as homogenizer), microfluidizer, high pressure extruder or the like, or subjected to freeze-thawing, to thereby disperse the lipid. Thus, a dispersion of enzyme-encapsulating lipid vesicles containing an aqueous enzyme solution as the internal aqueous phase can be obtained. When freeze-thaw was used, or when a combination of freeze-thaw and a high pressure extruder was used, the number of coat layers can be effectively reduced. This is preferable because it results in remarkable improvement in filter permeability and reduction of handling time. Generally, the particle size of the finally obtained lipid vesicle composition is often small compared to the size obtained at this stage. Therefore, in the present invention, it is preferable in terms of productivity (yield, etc.) also to adjust the particle size in advance taking into account of this side reduction. The particle size (number-average particle size) of the finally obtained lipid vesicle composition of the present invention is not particularly limited. In view of encapsulation efficiency, sterilization treatment and effect on hemodynamics, the particle size is preferably 30-450 nm, more preferably 80-250 nm. Subsequently, if necessary, vesicles are subjected to a method such as extrusion to give a desired particle size (number-average particle size) and particle size distribution. Then, unencapsulated enzyme is separated/removed by a method such as gel filtration, centrifugation (ultracentrifugation) or ultrafiltration, to thereby obtain the lipid vesicle composition of the present invention.

In the preparation method of the present invention, it is important to carry out the above-described formation of a lipid bilayer membrane in the presence of a pH buffer and/or a surfactant (more preferably, for example, in the presence of a pH buffer or in the presence of a pH buffer and a surfactant). By doing this, it is possible to obtain a lipid vesicle composition capable of stably retaining the activity of the encapsulated enzyme even outside of the stable pH range of the enzyme. Therefore, the present invention also encompasses a method for stabilizing an enzyme.

Specifically, a pH buffer and/or a surfactant may be allowed to be present in the preparation at least until the start of formation of a lipid bilayer membrane by association of the membrane-forming lipid. Generally, it is preferable to mix a pH buffer and/or a surfactant with an aqueous enzyme solution before, or simultaneously with, the addition of a membrane-forming lipid to the aqueous enzyme solution.

Examples of the pH buffer include, but are not limited to, glycine buffer, monopotassium phthalate buffer, succinate buffer, monopotassium citrate buffer, Tris(hydroxymethyl)aminomethane buffer, borate buffer, acetate buffer or the like may be enumerated as described earlier. Among all, monopotassium citrate buffer is preferable when the enzyme is glucocerebrosidase.

By using such a pH buffer, it is possible to appropriately adjust and set the pH of the aqueous enzyme solution depending on the stable pH range for the enzyme. For example, when glucocerebrosidase is to be encapsulated, the pH of the aqueous enzyme solution is preferably 2 to 7, more preferably 4 to 6.5. The volume of a pH buffer to be used is not particularly limited, and may be appropriately determined.

Preferable examples of the surfactant include, but are not limited to, polyoxyethylenesorbitan monooleate, polyoxyethylenesorbitan monolaurate, polyoxyethylenesorbitan monopalmitate, polyoxyethylenesorbitan monostearate, polyoxyethylenesorbitan trioleate or the like (such as polysorbate 80). By using such a surfactant in an appropriate amount, it is possible to produce an effect that leads to stabilization of the encapsulated enzyme without exerting a bad influence upon formation of lipid vesicles. It is intended that the term "surfactant" used in the present invention includes every compound belonging to emulsifier or solubilizer in general.

In the preparation method of the present invention, components such as various additives other than the above-described pH buffer and surfactant may be mixed in the formation of a lipid bilayer membrane. These components are not particularly limited.

In the preparation method of the present invention, in order to prevent denaturation (decrease of activity) of the encapsulated enzyme, it is generally preferable that processes handling the enzyme are performed at 20°C or below. However, the temperatures are not limited to this range.

In the preparation method of the present invention, a preferred embodiment is given in which a pH buffer and/or a surfactant is present in both the internal aqueous phase (internal liquid) and the external aqueous phase (external liquid) of the resultant lipid vesicle composition. Usually, according to the preparation method of the present invention, a pH buffer and/or a surfactant is present together with the enzyme in the internal aqueous phase of the resultant vesicles, and the pH buffer and/or the surfactant is also present in the external aqueous phase of the vesicles because these vesicles are formed in a solution containing the pH buffer and/or the surfactant. Therefore, to practice the preparation method of the present invention may inevitably result in the above-described preferred embodiment. Alternatively, the preferred embodiment may be achieved in a different manner. For example, in order to remove the unencapsulated enzyme, the resultant lipid vesicle composition may be once isolated and transferred into another solution to thereby replace the external aqueous phase of the vesicles with another solution (containing a pH buffer and/or a surfactant).

By employing the above-described preferred embodiment, it is possible to retain the encapsulated enzyme in a more stable state and to maintain its effective enzyme activity for a longer period. Thus, in long term storage of preparations to be used in enzyme replacement therapy and the like, it is possible to further enhance the stability of such preparations.

In the above-described preferred embodiment, various conditions (pH, concentration, type, etc.) of the pH buffers and the surfactants present in the external and internal aqueous phases may be either the same or different with each other. Such conditions may be determined appropriately.

### (5) Property of the Lipid Vesicle Composition

The lipid vesicle composition of the present invention has a property that it is capable of retaining stably the activity of the encapsulated enzyme even outside the stable pH range of the enzyme. For example, even when an enzyme that is stable and exerts activity in the acidic range (e.g., glucocerebrosidase) is placed in the neutral range where its activity decreases or it is deactivated, the enzyme is capable of retaining and exerting its activity equivalent to that in the acidic range if the enzyme is encapsulated in the lipid vesicle composition of the present invention.

With respect to the above-described property of "capable of retaining stably the activity of the enzyme", preferably, the property is defined as described in (a) below. However, the definition of the property is not limited to this.
(a) Under conditions that a lipid vesicle composition encapsulating an enzyme is transferred from the stable pH range of the enzyme to the outside thereof (unstable pH range) and left there for two hours, the remaining ratio of enzyme activity (based on the enzyme activity in the stable pH range) is preferably 80% or more, more preferably 90% or more. It should be noted here that these numerical ranges are ranges given for cases where the encapsulated enzyme *per se* shows a remaining ratio of enzyme activity of 80% or less under the above-described conditions.

Since the lipid vesicle composition of the present invention has the above-described property concerning the stability of enzyme activity, the composition is capable of effectively functioning as the so-called enzyme carrier that supplements an enzyme of interest (the enzyme encapsulated in lipid vesicles) to a target organ in the living body through blood flow Therefore, as described below, the lipid vesicle composition of the present invention is extremely useful in therapeutics agent or treatment methods for diseases that need enzyme replacement.

### 2. Therapeutic Agent and Treatment Method

### (1) Therapeutic agent

The therapeutic agent of the present invention is, as described above, a therapeutic agent (pharmaceutical composition, therapeutic composition) for treating diseases caused by enzyme deficiency or decrease in enzyme activity and characterized by comprising the above-described lipid vesicle composition of the present invention. Further, the present invention also encompasses use of the above-described lipid vesicle composition in the preparation of a therapeutic agent for treating diseases caused by enzyme deficiency or decrease in enzyme activity.

Diseases caused by enzyme deficiency or decrease in enzyme activity may be either congenital lysosomal diseases. Specifically, Gaucher disease may be given. When the above disease is Gaucher disease, it is preferred that glucocerebrosidase be indispensable as the enzyme to be encapsulated in the lipid vesicle composition included in the therapeutic agent of the present invention.

Generally, the therapeutic agent of the present invention is preferably a dispersion obtained by dispersing the lipid vesicle composition of the invention in an appropriate dispersion medium, so that intravenous infusion or the like is possible when the therapeutic agent is administered into the living body.

The pH of the dispersion medium may be within a range that is acceptable as physiological pH, and may be set appropriately within a range where no remarkable influence occurs in the stability of the lipid vesicle composition or in the state of the assembly of vesicles. The physiological pH range means the pH range within the living body, the blood, etc. For example, the physiological pH range is pH 4-11, preferably pH 6-9, more preferably pH 7-8 (almost neutral).

In the therapeutic agent of the present invention, the ratio of the lipid vesicle composition in the above-described dispersion medium may be appropriately selected depending on the type and the progress of the target disease, the condition of the patient, and the type of the enzyme to be supplemented.

The therapeutic agent of the present invention may contain components other than the lipid vesicle composition and the dispersion medium. Such components are not particularly limited. For example, excipients, surfactants, dispersants, buffers, preservatives, dissolution aids, antiseptics, flavoring agents, anesthetics, stabilizers, isotonizing agents and the like which are commonly used in preparation of drugs may be enumerated. Further, when the therapeutic agent of the present invention is formulated in various injections, aliphatic polyalcohols such as glycerol, polypropylene glycol and polyethylene glycol may be contained. These components may be combined and prepared in the therapeutic agent by conventional methods.

When the therapeutic agent of the present invention is used as a parenteral agent such as an injection, generally the form thereof is not particularly limited. For example, the therapeutic agent may be any one of intravenous injection (including infusion), intramuscular injection, intraperitoneal injection and subcutaneous injection. When the therapeutic agent is various injections, they may be supplied, for example, in unit dose ampules or multi-dose containers, or in the form of freeze-dried powder which is re-dissolved in solution at the time of use.

### (2) Treatment Method

The treatment method of the present invention for diseases caused by enzyme deficiency or decrease in enzyme activity is, as described earlier, characterized by administering the lipid vesicle composition of the present invention to patients with such diseases. Further, the present invention encompasses use of the lipid vesicle composition of the present invention for treating diseases caused by enzyme deficiency or decrease in enzyme activity. It can be said that the administration or use of the lipid vesicle composition is substantially administration or use of the therapeutic agent of the present invention.

Lysosomal diseases are congenital diseases caused by enzyme deficiency or decrease in enzyme activity. Specifically, Gaucher disease may be given preferably. When the above disease is Gaucher disease, it is preferred that glucocerebrosidase is indispensable as the encapsulated enzyme in the lipid vesicle composition contained in the therapeutic agent of the present invention.

In the treatment method of the present invention, the method of administration into patients with the above-described diseases is not particularly limited. Generally, known parenteral methods may be preferable. Specifically, intravenous injection (including infusion), intramuscular injection, intraperitoneal injection, subcutaneous injection and the like may be enumerated. Intravenous injection is preferably used.

The dose, administration time, administration intervals and the number of administration (/day) may be appropriately determined considering the ratio of the active ingredient (enzyme) contained in the preparation (therapeutic agent of the present invention) and taking into account of the age, body weight and condition of the patient and the type and the progress of the target disease. However, in enzyme replacement therapy according to the treatment method of the present invention, the enzyme is used being encapsulated in lipid vesicles, as described earlier. As a result, the stability in blood and the targeting property of the enzyme are very high compared to conventionally used enzymes. This produces remarkable improvement effects such as reduction of dose and administration time, prolongation of administration intervals, etc.

Hereinbelow, the present invention will be described more specifically with reference to the following Examples. However, the scope of the present invention is not limited to these Examples.

### [EXAMPLE 1]

### Preparation and Evaluation of Glucocerebrosidase-Encapsulating Liposome Preparation

### 1. Methods

### 1-1 Preparation of Glucocerebrosidase-Encapsulating Liposome Preparation

Mixed lipid (total 70 mg; 1,2-dipalmitoyl-*sn*-glycero-3-phosphatidylcholine/ cholesterol/ 1,5-*O*-dihexadecyl-*N*-succinyl-L-glutamate (DHSG)/ *N*-[methoxypolyethyleneglycol(5000)-carbamyl]distearoyl-*sn*-glycero-3-phosphatidylethanolamine = 5/5/1/0.033 (molar ratio)) was hydrated with a glucocerebrosidase solution (2 mg/mL, 2 mL, 0.028% polysorbate 80, 100 mM citrate buffer (pH6.0)), agitated and left at room temperature for 1 hour. Then, the particle size was regulated by extrusion (ϕ: 0.8, 0.6, 0.45 and 0.22 µm).

Unencapsulated glucocerebrosidase was removed by centrifugation (100,000 g, 30 minutes, 4°C) to thereby obtain a glucocerebrosidase-encapsulating liposome preparation (glucocerebrosidase/liposome preparation) as an enzyme-encapsulating lipid vesicle composition (lipid concentration = 19.3 mg/mL; 1.7 mL). As a dispersion media for this liposome preparation, 0.014% polysorbate 80-containing 100 mM citrate buffer (pH 6.0) was used.

### 1-2 Enzyme Activity Measurement Method

The enzyme preparation (10 µL) was mixed with 10µL of 160 mM citrate buffer (pH 6.0) containing 2% polyoxyethylene 10 lauryl ether and 0.014% polysorbate 80, and was heated in water bath at 50°C for 40 seconds to solubilize the liposome, and then immediately ice-cooled. To the resultant solution, 4 mM 4-methylumbelliferyl β-D-glucopyranoside and 0.8% sodium taurocholate-containing 0.2 M acetate buffer (pH 5.5, 60 µL) were added and mixed. The resultant mixture was incubated at 37 °C for 30 minutes. 0.2 M glycine buffer (pH 10.7, 700 µL) was added thereto to quench the reaction and the fluorescence of liberated 4-methylumbelliferon (4MU) was measured (Eₓ: 365 nm; Eₘ: 450 nm).

Using the separately prepared calibration curve of 4MU, the concentration of free 4MU was calculated. Then, the quantity of 4MU that 1 mL of the enzyme solution can liberate per hour was taken as enzyme activity in solution. The value obtained by dividing this quantity of 4MU by protein concentration (unit: mg/mL) was taken as enzyme activity in tissue.

### 1-3 Experimental Animals and Group Constitution

C57BL/6 CrSLC, female, 12-week old mice were used in groups each consisting of three individuals. The group constitution is shown below.
1) Administration of free form enzyme/24 hr evaluation
2) Administration of liposome preparation/24 hr evaluation
3) Administration of free form enzyme/2 hr evaluation
4) Administration of liposome preparation/2 hr evaluation
5) Control (untreated)

### 1-4 Administered Preparations and Doses

Dose of the free form enzyme was set at 1 mg/kg. With respect to the liposome preparation, a dose which gives the same enzyme activity value as that of this amount of free form enzyme was administered.

Each preparation was filtered through a 0.45 µm sterilization filter, followed by measurement of enzyme activity. Then, each preparation was diluted with 0.014% polysorbate 80-containing 100 mM citrate buffer (pH 6.0) pre-filtered through a sterilization filter to make the enzyme activity 135 µmol/hr/mL. Each dose was calculated taking the body weight of mouse as 25 g and the dose volume as 0.2 mL/head.
- Free form enzyme: 1.08 mmol/hr/kg BW = 1.0 mg/kg BW
- Liposome preparation: 1.08 mmol/hr/kg BW = 2.8 mg/kg BW

### 1.5 Administration, Collection of Blood and Removal of Tissues

Using a 1 mL tuberculin syringe provided with 1/5 hypodermic needle, the above-mentioned administration solution (200 µL) was bolus administered to each mouse from the tail vein. Points of blood collection were as follows.
- 24 hr evaluation groups: 3, 10, 30 min and 1, 2, 3, 4, 24 hr after the completion of administration
- 2 hr evaluation groups: 3, 10, 30 min and 1, 2 hr after the completion of administration

A blood sample (approx. 25 µL) was taken from each mouse from the tail with a hematocrit tube and centrifuged to obtain a plasma fraction, which was immediately cryopreserved at -80°C. The final blood collection was performed under etherization from the orbit, and a plasma sample was obtained in the same manner as described above. After completion of the blood collection, mice were euthanized by cervical spine dislocation and decapitation, followed by removal of the spleen, liver and kidney. These organs were washed with PBS(-) twice, quickly frozen with liquid nitrogen and cryopreserved at -80°C.

### 1-6 Measurements of Plasma and Tissue Enzyme Activities

The plasma sample, as it was or appropriately diluted with 0.014% polysorbate 80-containing 50 mM citrate buffer (pH 6.0), was subjected to enzyme activity measurement as described above under the item "Enzyme Activity".

With respect to the spleen, a 19-fold volume (relative to the weight of the tissue) of 0.014% polysorbate 80-containing 50 mM citrate buffer (pH 6.0) was added. With respect to the liver and kidney, a 4-fold volume of 0.014% polysorbate 80-containing 50 mM citrate buffer (pH 6.0) was added individually. Then, homogenate was prepared with Polytron and sonicated (for 5 seconds). Spleen homogenate, as it was, was subjected to enzyme activity measurement. Liver and Kidney homogenates were subjected to enzyme activity measurement after 10-fold dilution with 0.014% polysorbate 80-containing 50 mM citrate buffer (pH 6.0). Protein concentrations were also measured by the Bradford method.

### 1-7 Western Blotting

The sonicated spleen homogenate which was prepared for measuring tissue enzyme activity was centrifuged at 12,000 rpm for 5 minutes. The resultant supernatant was used as a sample. To the protein extracted therefrom (2.5 mg), Con A Sepharose (30 µL) was added, and was incubated overnight at 4 °C for binding of the protein. The resin was washed with 1% Triton X-100/TBS, mixed with SDS-PAGE sample buffer, and then boiled. The sample was subjected to SDS-PAGE. Subsequently, Western blotting was performed using anti-glucocerebrosidase antibody.

### 1-8 Calculation ofPK Parameters

By non-compartment analysis using WinNonlin ver. 4.1, time to attain maximum plasma enzyme activity (Tₘₐₓ), maximum plasma enzyme activity (Cₘₐₓ), area under the plasma enzyme activity-time curve (AUC) and clearance half-time (t_{1/2}) were obtained.

### 2. Results

### 2-1 Specification of Preparations

The specification of preparations is given in Table 2 below.

The encapsulation efficiency was 17.4%; the particle size was 260±60 nm; and the lipid concentration was 19.3 mg/mL when the liposome preparation was formulated. Although about 50% decrease in specific activity was observed in the process of formulation, it was judged that this decrease is not a problem.

**Table 2**

| Specification of Preparations | | |
|---|---|---|
| | Free Form | Liposome Preparation |
| Particle size (nm) | | 260±60 |
| Lipid concentration (mg/mL) | | 19.3 |
| Protein concentration (mg/mL) | 2 | 0.41 |
| Encapsulation efficiency (%) | | 17.4 |
| Specific activity (µmol/hr/mg protein) | 810 | 390 |

### 2. Transition in Plasma Enzyme Activity and PK Parameters

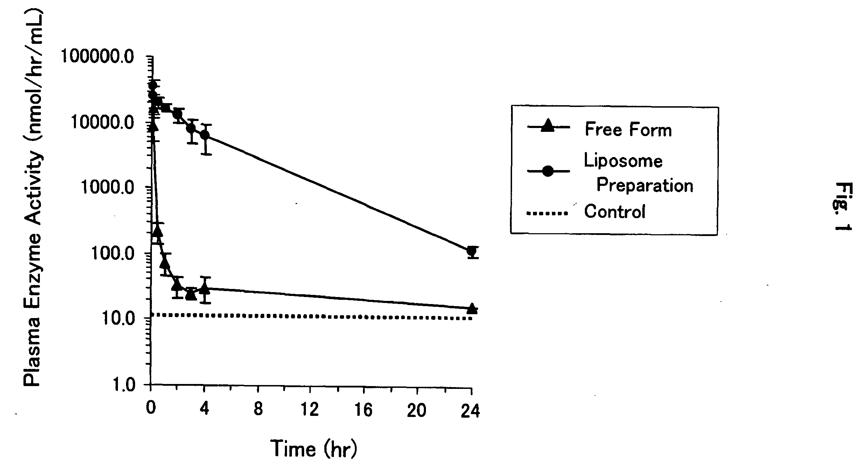

Plasma enzyme activity and PK parameters are shown in Table 3 below, and transition in average plasma enzyme activity is shown in Fig. 1.

The enzyme activity in plasma of C57BL 6 mice was 11.7 nmol/hr/mL.

After intravenous administration of glucocerebrosidase (free form), plasma enzyme activity decreased extremely rapidly. Average enzyme activities at immediately after the administration, 2 hours and 24 hours after the administration were 16.1 µmol/hr/mL, 31.9 nmol/hr/mL and 16.0 nmoUhr/mL, respectively. Average AUC24hr was 4.79 µmol/mL.

On the other hand, when the liposome preparation was administered, plasma enzyme activity decreased slowly compared to the administration of the free form. Average enzyme activities at immediately after the administration, 2 hours and 24 hours after the administration were 34.5 µmol/hr/mL, 13.2 µmol/hr/mL and 114 nmol/hr/mL, respectively. Average AUC24hr was 116 µmol/mL.

One case in the liposome administration/2 hr group had imperfect administration. Thus, the data thereof was not adopted. Besides, at the first blood collection point, blood collection time was varied. The average value, 3.8 min, was taken to prepare the graphs. For calculation of PK parameters, actual values were used.

**Table 3**

| Plasma Enzyme Activity and PK Parameters after Administration of Glucocerebrosidase in Free Form and Liposome Preparation | | | | | | | | | | |
|---|---|---|---|---|---|---|---|---|---|---|
| Administered | | Time | Plasma Enzyme Activity (nmol/hr/mL) | | | | | | | |
| Preparation | | (hr) | 1 | 2 | 3 | 4 | 5 | 6 | Mean | SD |
| *Control* | | | | | | | | | | |
| | | | 9.8 | 12.0 | 13.4 | | | | 11.7 | 1.8 |

| *Free* | | | | | | | | | | |
|---|---|---|---|---|---|---|---|---|---|---|
| | | 0.064 | 18780.0 | 9160.0 | 15220.0 | 13500.0 | 22680.0 | 17340.0 | 16113.3 | 4641.4 |
| | | 0.167 | 7560.0 | 4280.0 | 8660.0 | 16280.0 | 8500.0 | 8040.0 | 8886.7 | 3963.8 |
| | | Q5 | 141.6 | 157.0 | 230.2 | 295.8 | 294.8 | 138.2 | 209.6 | 74.3 |
| | | 1 | 40.6 | 82.4 | 90.0 | 103.8 | 62.2 | 43.2 | 70.4 | 25.8 |
| | | 2 | 264 | 31.4 | 20.2 | 48.4 | 40.0 | 25.2 | 31.9 | 10.5 |
| | | 3 | | | | 272 | 27.2 | 18.6 | 24.3 | 5.0 |
| | | 4 | | | | 20.4 | 26.0 | 43.8 | 30.1 | 12.2 |
| | | 24 | | | | | 8.4 | 23.6 | 16.0 | 10.7 |

| | PK parameter | | | | | | | | | |
|---|---|---|---|---|---|---|---|---|---|---|
| | Tmax | (hr) | 0.06 | 0.10 | 0.07 | 0.19 | 0.04 | 0.05 | 0.09 | 0.06 |
| | Omax | (nmol/hr/mL) | 18780.0 | 9160.0 | 15220.0 | 16280.0 | 22680.0 | 17340.0 | 16576.7 | 4463.7 |
| | AUC2hr | (nmol/mL) | 4324.2 | 3322.2 | 4107.3 | | | | 3917.9 | 527.1 |
| | AUC4hr | (nmol/mL) | | | | 5580.1 | | | 5580.1 | NC |
| | AUC24hr | (nmol/mL) | | | | | 5010.8 | 4567.3 | 4789.0 | NC |
| | t1/2(2hr) | (hr) | 0.7 | 0.7 | 0.4 | | | | 0.6 | 0.1 |
| | t1/2(4hr) | (hr) | | | | 1.6 | | | 1.6 | NC |
| | t1/2(24hr) | (hr) | | | | | 12.3 | 22.4 | 17.4 | NC |

| *Liposome* | | | | | | | | | | |
|---|---|---|---|---|---|---|---|---|---|---|
| | | 0.064 | | 34160.0 | 48140.0 | 26080.0 | 32720.0 | 31440.0 | 34508.0 | 8209.6 |
| | | 0.167 | | 37360.0 | 29620.0 | 12780.0 | 24360.0 | 22880.0 | 25400.0 | 9049.1 |
| | | 0.5 | | 22520.0 | 19800.0 | 14360.0 | 20400.0 | 24140.0 | 20244.0 | 3714.9 |
| | | 1 | | 17400.0 | 19220.0 | 13380.0 | 16940.0 | 14840.0 | 16356.0 | 2279.7 |
| | | 2 | | 9880.0 | 15900.0 | 12820.0 | 17260.0 | 10060.0 | 13184.0 | 3346.6 |
| | | 3 | | | | 8634.0 | 10494.0 | 4442.0 | 7856.7 | 3100.0 |
| | | 4 | | | | 7398.0 | 8614.0 | 28820 | 6298.0 | 3020.2 |
| | | 24 | | | | 117.2 | 134.2 | 91.2 | 114.2 | 21.7 |

| | PK parameter | | | | | | | | | |
|---|---|---|---|---|---|---|---|---|---|---|
| | Tmax | (hr) | | 0.18 | 0.07 | 0.06 | 0.04 | 0.04 | 0.08 | 0.06 |
| | Qmax | (nmol/hr/mL) | | 37360.0 | 48140.0 | 26080.0 | 32720.0 | 31440.0 | 35148.0 | 8300.0 |
| | AUC2hr | (nmol/mL) | | 39490.9 | 43450.3 | | | | 41470.6 | 2799.7 |
| | AUC24hr | (nmol/mL) | | | | 122630.8 | 149813.9 | 75443.9 | 115962.9 | 37630.7 |
| | t1/2(2hr) | (hr) | | 1.3 | 4.5 | | | | 2.9 | 2.3 |
| | t1/2(24hr) | (hr) | | | | 3.4 | 3.3 | 3.9 | 3.5 | 0.3 |

### 2-3 Tissue Enzyme Activities

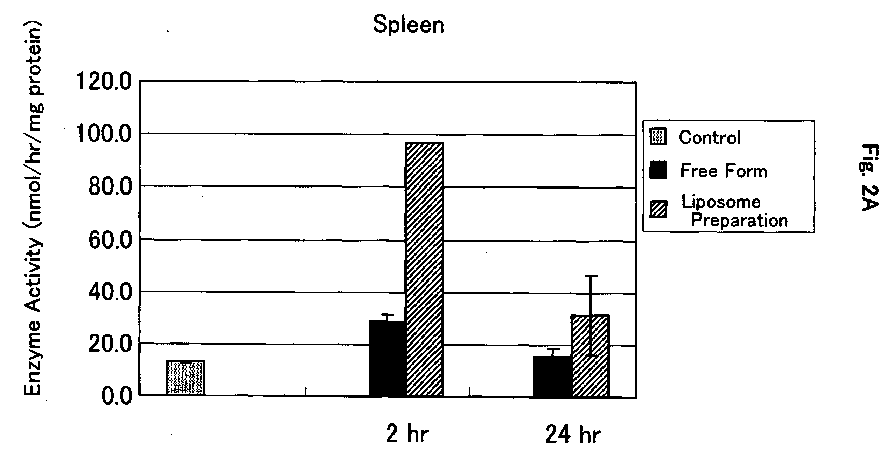

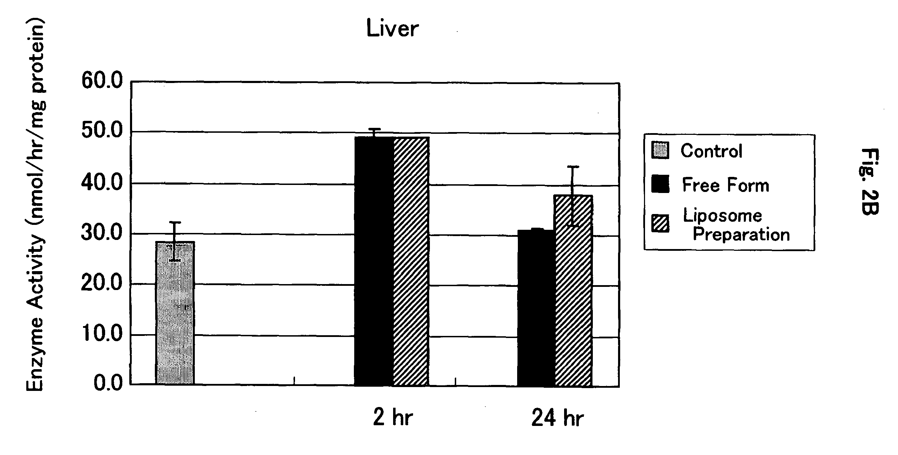

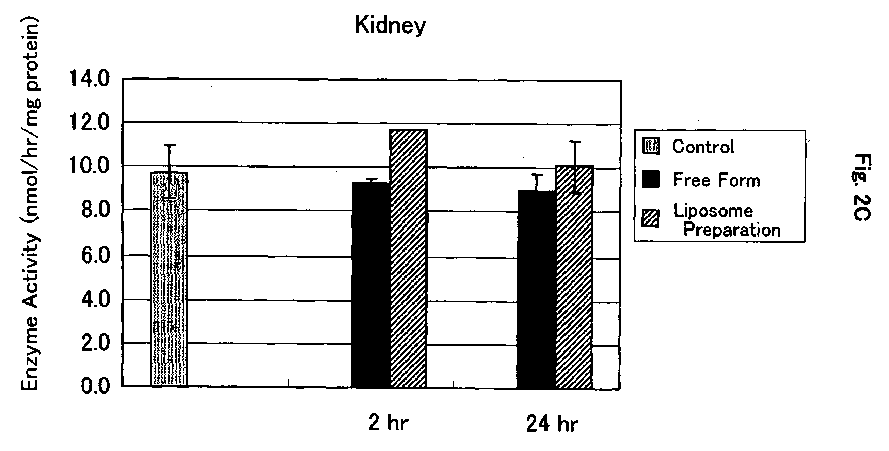

Tissue enzyme activities are shown in Table 4 below and Fig. 2A-2C.

In the kidney (a non-target organ), increase of enzyme activity was little when each preparation was administered. Enzyme uptake was extremely small.

The enzyme activities in the spleen were 13.2 nmol/hr/mg protein in control group; and 28.2 and 15.2 nmol/hr/mg protein at 2 hours and 24 hours after the administration of free form, respectively. In liposome preparation administration group, enzyme activities were 97.6 and 31.2 nmol/hr/mg protein at 2 hours and 24 hours, respectively.

The enzyme activities in the liver were 28.5 nmol/hr/mg protein in control group; and 49.0 and 30.8 nmol/hr/mg protein at 2 hours and 24 hours after the administration of free form, respectively. In liposome preparation administration group, enzyme activities were 51.4 and 37.9 nmol/hr/mg protein at 2 hours and 24 hours, respectively.

**Table 4**

| Tissue Enzyme Activities after Administration of Glucocerebrosidase in Free Form and Liposome Preparation | | | | | | | |
|---|---|---|---|---|---|---|---|
| Tissue | Administered | Time | Tissue Enzyme Activity (nmol/hr/mg protein) | | | | |
| | Preparation | (hr) | 1 | 2 | 3 | Mean | SD |
| Spleen | Control | | 13.2 | 13.5 | 12.9 | 13.2 | 0.3 |
| | Free | 2 | 30.5 | 25.2 | 29.0 | 28.2 | 2.7 |
| | | 24 | 18.6 | 14.9 | 12.1 | 15.2 | 3.3 |
| | Liposome | 2 | | 91.0 | 104.2 | 97.6 | NC |
| | | 24 | 24.6 | 48.4 | 20.5 | 31.2 | 15.1 |
| Liver | Control | | 24.2 | 30.7 | 30.6 | 28.5 | 3.7 |
| | Free | 2 | 50.4 | 49.6 | 47.1 | 49.0 | 1.7 |
| | | 24 | 30.5 | 30.6 | 31.4 | 30.8 | 0.5 |
| | Liposome | 2 | | 50.2 | 52.6 | 51.4 | NC |
| | | 24 | 40.4 | 42.0 | 31.3 | 37.9 | 5.8 |
| Kidney | Control | | 11.1 | 9.1 | 9.0 | 9.7 | 1.2 |
| | Free | 2 | 9.5 | 9.1 | 9.2 | 9.3 | 0.2 |
| | | 24 | 9.6 | 8.1 | 9.1 | 8.9 | 0.8 |
| | Liposome | 2 | | 12.3 | 12.2 | 12.3 | NC |
| | | 24 | 8.9 | 10.0 | 11.3 | 10.1 | 1.2 |

### 2-4 Western Blotting

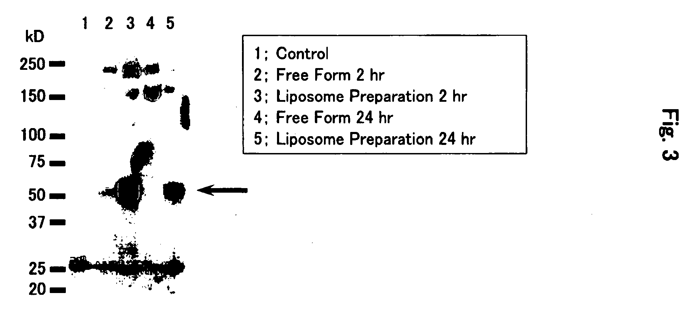

It is believed that the content ratio of target cells to the whole organ is the highest in the spleen. Therefore, spleen was used for evaluation. Protein extracted from spleen homogenate was subjected to Western blotting. In the control group, almost no band was detected around the molecular weight of glucocerebrosidase (approx. 60 kD). In the free form administration groups, bands corresponding to glucocerebrosidase were detected slightly at 2 hours after the administration, but they were undetectable at 24 hours. On the other hand, in the liposome preparation administration groups, very high concentration of the protein could be detected at 2 hours after the administration; even at 24 hours, still higher concentration than that detected at 2 hours after the administration of free form could be detected (Fig. 3).

### [EXAMPLE 2]

### Confirmation of Localization of Glucocerebrosidase-Encapsulating Liposome by Immunostaining

### 1. Materials and Methods

### 1-1 Preparation of Glucocerebrosidase-Encapsulating Liposome

A preparation in which glucocerebrosidase is encapsulated in liposome (glucocerebrosidase-encapsulating liposome preparation) was prepared in the same manner as described in Example 1.

### 1-2 Preparation of Cryosections

Cryosections were prepared by conventional methods from mouse liver, spleen and kidney which had been removed 2 hours after the administration of the glucocerebrosidase-encapsulating liposome preparation.

Cryosections were prepared with a cryostat, attached to slide glass and dried sufficiently.

### 1-3 Immunostaining

The cryosections attached to slide glass was fixed with 4% paraformaldehyde PBS(-) solution for 30 minutes. After washing 3 times with PBS(-), the cryosections was subjected to blocking treatment in 1% BSA-containing PBS(-) solution for 1 hour. After washing with PBS(-), the cryosections was reacted with anti-rabbit glucocerebrosidase serum and rat anti-macrophage antibody (as primary antibodies) in the presence of 1% BSA at room temperature for 1 hour. After washing 3 times with 0.3% polysorbate 20-containing PBS(-) solution, the cryosections was reacted with anti-rabbit Alexa488 and rat rhodamine (as secondary antibodies) in the presence of 1% BSA at room temperature for 1 hour. After washing 3 times with 0.3% polysorbate 20-containing PBS(-) solution, the cryosections was mounted with a mounting medium.

### 1-4 Fluorescence Observation

Fluorescently stained mouse liver tissue sections were observed with a confocal laser microscope.

### 2. Results

The staining of liver Kupffer cells could be confirmed in mouse liver sections (Fig. 5; the portions stained with red color in panels in the upper, the middle and the bottom row). In the liver of mice to which the glucocerebrosidase-encapsulating liposome was administered, hypertrophy of Kupffer cells (Fig. 5; the portion stained with red color in panels in the upper row) could be confirmed, compared to Kupffer cells in the free form and physiological saline administration groups (Fig. 5; the portions stained with red color in panels in the middle and the bottom row).

The staining of glucocerebrosidase could be confirmed in both the liver of mice to which glucocerebrosidase was administered and the liver of mice to which the glucocerebrosidase-encapsulating liposomes were administered (Fig. 5; the portions stained with green color in panels in the upper and the middle row). It was widely observed that fluorescence transferred to parenchymal cells in the liver of mice to which glucocerebrosidase was administered. Whereas, fluorescence was selectively localized to Kupffer cells in the liver of mice to which the glucocerebrosidase-encapsulating liposomes were administered. In the liver of mice to which physiological saline was administered, no glucocerebrosidase-derived fluorescence was observed (Fig. 5; the middle panel in the bottom row).

It was confirmed by superimposition of both fluorescent images detecting the localization of macrophage and the one of glucocerebrosidase are consistent in the liver of mice to which the glucocerebrosidase-encapsulating liposomes were administered (Fig. 5; the right panel in the upper row).

From what have been described above, it was demonstrated that the enzyme-encapsulating liposome of the present invention is excellent in transfer to a target organ (liver in this Example) and useful in enzyme replacement therapy.

### [Discussion]

After intravenous administration of glucocerebrosidase in a free form, the plasma enzyme activity decreased extremely rapidly. Although the activity was 2.7-fold higher than the control at 2 hours after the administration, it decreased to 1.4-fold higher at 24 hours. On the other hand, the plasma enzyme activity after administration of the liposome preparation decreased slowly compared to the administration of the free form. The activity was 1124-fold higher than the control at 2 hours after the administration; even at 24 hours, 10-fold higher activity was maintained. When plasma enzyme activity ratio of liposome preparation/free form was calculated, the ratio was 413 at 2 hours after the administration and 7 at 24 hours after the administration. When compared in AUC24hr value which is an indicator of exposure, the value for liposome preparation was 24-fold higher than that of free form.

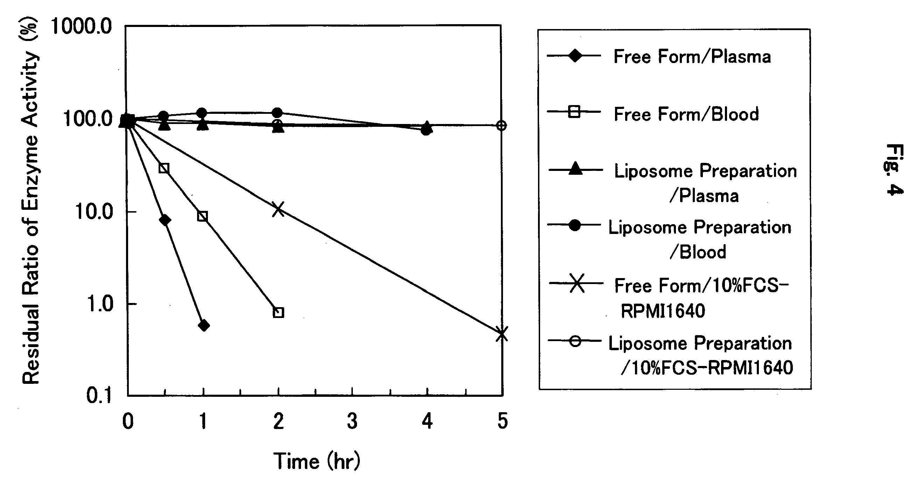

Although lysosomal enzymes are stable at weakly acidic pH within lysosomes, they are unstable under neutral conditions such as in plasma. Prior to this Example, the present inventors have also examined the stability of glucocerebrosidase in human plasma. As a result, it was confirmed that this enzyme is extremely unstable in its free form, however it can be stabilized by formulating into a liposome preparation such as described in the Example (Fig. 4). It is believed that such stabilization is achieved by isolating the enzyme from the neutral environment (plasma). Based upon this finding, the present inventors have also found in this Example that the enzyme can be stabilized similarly in mouse *in vivo*, and succeeded in increasing AUC.

As a result of improvement in plasma kinetics, delivery to the target organ has also increased.

In the spleen, while the enzyme activity at 2 hours after the administration of free form was 2.1-fold higher than the control, the activity was 7.4-fold higher when liposome was administered. Furthermore, at 24 hours after the administration of free form, the activity decreased to the background level, whereas 2.4-hold higher activity than control could be maintained at 24 hours after the administration of liposome.

In the liver, a little less than 2-fold activity increase was observed at 2 hours after the administration of both preparations. However, at 24 hours, the activity was the background level. It seems that uptake to the liver is smaller than uptake to the spleen. However, it is believed that a sufficient amount has been taken into target cells because of the following reasons: the ratio of Kupffer cells (target cells; the liposome preparation is believed to be selectively taken into these cells) in the total liver cells is about 15%; and generally, most of free form is taken into hepatic parenchymal cells.

In order to clarify the above point, liver sections were immunostained. The results revealed that the glucocerebrosidase-encapsulating liposome is selectively taken into Kupffer cells in the liver (the target cell) (Fig. 5).

From the results obtained at this time, it was confirmed that more effective delivery to a target tissue (cell) can be achieved by formulating glucocerebrosidase into a liposome preparation. As one factor, improvement of residence time in blood may be given. That is, while most part of administered enzyme loses its activity before it is taken into the target tissue when a free form enzyme is administered, a liposome preparation that acquired stability in blood is circulating in the body in an active state. It can be concluded that this liposome preparation was gradually and effectively taken into the target tissue.

### INDUSTRIAL APPLICABILITY

According to the present invention, it is possible to provide a lipid vesicle composition excellent in stability in blood and transfer to its target organ. A therapeutic agent for Gaucher disease comprising the above composition as an active ingredient can also be provided. Further, a method for preparing the above composition, a method for stabilizing an enzyme, and a method for treating Gaucher disease can also be provided.

## Claims

1. A lipid vesicle composition wherein vesicles composed of a lipid bilayer membrane encapsulate therein a lysosomal enzyme relating to lysosomal disease,
wherein the lipid bilayer membrane contains diacylphosphatidylcholine, cholesterol, 1,5-*O*-dihexadecyl-*N*-succinyl-L-glutamate, and a polyethylene glycol (PEG) chain-linked lipid, wherein the molecular weight of the PEG chain is 2000-5000, and
wherein the lysosomal enzyme is in aqueous solution including a pH buffer, wherein the pH of the aqueous solution is within the stable pH range of the enzyme.

2. The composition according to claim 1, wherein the diacylphosphatidylcholine is dipalmitoylphosphatidylcholine.

3. The composition according to claim 1, wherein the lipid bilayer membrane contains 1,2-dipalmitoyl-sn-glycero-3-phosphatidylcholine, cholesterol, 1,5-*O*-dihexa-decyl-*N-*succinyl-L-glutamate and *N*-[methoxypolyethyleneglycol(5000)-carbamyl]distearoyl-sn-glycero-3-phosphatidyl-ethanolamine at a molar ratio of 5:5:1:0.033.

4. The composition according to any preceding claim, which is capable of functioning as an enzyme carrier that delivers the enzyme into Kupffer cells in the liver in the living body through blood flow.

5. The composition according to any one of claims 1 to 3, wherein said enzyme is glucocerebrosidase.

6. The composition according to claim 5, for use in a method of treating Gaucher disease.

7. Use of the composition according to claim 5 in the manufacture of a medicament for treating Gaucher disease.

## Patentansprüche

1. Lipidvesikelzusammensetzung, worin in aus einer zweischichtigen Lipidmembran bestehenden Vesikeln ein lysosomales Enzym in Bezug auf lysosomale Erkrankung eingeschlossen ist,
worin die zweischichtige Lipidmembran Diacylphosphatidylcholin, Cholesterin, 1,5-O-Dihexadecyl-N-succinyl-L-glutamat und ein an eine Polyethylenglykol- (PEG-) Kette gebundenes Lipid enthält, worin das Molekulargewicht der PEG-Kette 2.000 bis 5.000 beträgt und
worin das lysosomale Enzym in wässriger, einen pH-Puffer enthaltender Lösung vorliegt, worin der pH-Wert der wässrigen Lösung im stabilen pH-Bereich des Enzyms liegt.

2. Zusammensetzung nach Anspruch 1, worin das Diacylphosphatidylcholin Dipalmitoylphosphatidylcholin ist.

3. Zusammensetzung nach Anspruch 1, worin die zweischichtige Lipidmembran 1,2-Dipalmitoyl-sn-glycero-3-phosphatidylcholin, Cholesterin, 1,5-O-Dihexadecyl-N-succinyl-L-glutamat und N-[Methoxypolyethylenglykol(5000)carbamyl]distearoyl-sn-glycero-3-phosphatidylethanolamin in einem Molverhältnis von 5:5:1:0,033 enthält.

4. Zusammensetzung nach einem der vorangegangenen Ansprüche, die in der Lage ist, als Enzymträger zu wirken, der das Enzym Kupffer-Zellen in der Leber im lebenden Körper durch den Blutstrom zuführt.

5. Zusammensetzung nach einem der Ansprüche 1 bis 3, worin das Enzym Glucocerebrosidase ist.

6. Zusammensetzung nach Anspruch 5 zur Verwendung in einem Verfahren zur Behandlung von Morbus Gaucher ist.

7. Verwendung einer Zusammensetzung nach Anspruch 5 bei der Herstellung eines Medikaments zur Behandlung von Morbus Gaucher.

## Revendications

1. Composition de vésicule lipidique, dans laquelle des vésicules constituées d'une membrane bicouche lipidique encapsulent dans celle-ci une enzyme lysosomiale liée à une maladie lysosomiale,
où la membrane bicouche lipidique contient du diacylphosphatidylcholine, cholestérol, 1,5-0-dihexadéyl-N-succinyle-L-glutamate, et un lipide lié à la chaîne de polyéthylène glycol (PEG), où le poids moléculaire de la chaîne PEG est 2000-5000, et
où l'enzyme lysosomiale est dans une solution aqueuse incluant un tampon pH, où le pH de la solution aqueuse est dans la page de pH stable de l'enzyme.

2. Composition selon la revendication 1, dans laquelle la diacyl-phosphatidyl-choline est la dipalmitoyl-phosphatidyl-choline.

3. Composition selon la revendication 1, dans laquelle la membrane bicouche lipidique contient 1,2-dipalmitoyl-sn-glycéro-3-phosphatidylcholine, cholestérol, 1,5-*0*-dihexa-décyl-N-succinyl-L-glutamate et *N*-[méthoxypolyéthylène-glycol (5000) carbamyl]distéaroyl-sn-glycéro-3-phosphatidyl-éthanolamine à un rapport molaire de 5:5:1:0,033.

4. Composition selon l'une quelconque des revendications précédentes, qui est apte à fonctionner comme un support d'enzyme qui délivre l'enzyme dans des cellules de Kupffer dans le foie dans le corps vivant par le flux sanguin.

5. Composition selon l'une quelconque des revendications 1 à 3, dans laquelle ladite enzyme est la glucocérébrosidase.

6. Composition selon la revendication 5, pour utilisation dans une méthode de traitement de la maladie de Gaucher.

7. Utilisation de la composition selon la revendication 5 dans la fabrication d'un médicament pour le traitement de la maladie de Gaucher.
